# EUROPEAN PATENT SPECIFICATION

(11) **EP 1 867 699 B1**
(45) Date of publication and mention of the grant of the patent: **10.10.2018**
(21) Application number: 05727778.2
(22) Date of filing: 31.03.2005
(51) Int. Cl.: C09K 15/04, A23L 3/3463, C08L 101/00, A23B 4/20, A23L 3/3472, A23L 3/3508, A23L 3/3526, A23L 3/3562, A61K 8/60, A61K 8/37, A61K 8/67, A61Q 19/00, C09K 15/06, C09K 15/34, A23C 3/08, A61K 8/49

(54) **DETERIORATION PREVENTING AGENT**
MITTEL ZUR VERHINDERUNG EINER VERSCHLECHTERUNG
AGENT DE PREVENTION DE LA DETERIORATION

(43) Date of publication of application: 19.12.2007
(73) Proprietor: Mitsubishi Chemical Corporation, Tokyo 100-8251 (JP)
(72) Inventor: KIDO, Hirotsugu, MITSUBISHI CHEMICAL CORPORATION, Yokohama-shi, Kanagawa 2278502 (JP)
(74) Representative: Hoffmann Eitle
(86) International application number: PCT/JP2005/006281
(87) International publication number: WO 2006/106573

(56) References cited:
- EP-A1- 1 183 954
- WO-A1-2004/020552
- JP-A- 8 253 764
- JP-A- 11 276 114
- JP-A- 2000 336 354
- JP-A- 2001 342 489
- JP-A- 2002 142 673
- JP-A- 2003 511 549
- US-A- 5 866 179
- US-A1- 2004 047 957
- US-A1- 2004 131 709
- Hisashi Uedaira et al.: "Hydration of oligosaccharides", Bulletin of Chemical society of Japan, vol. 63, no. 12 1 January 1990 (1990-01-01), 1990, pages 3376-3379, XP055044920, Retrieved from the Internet: URL:http://pdf.lookchem.com/pdf/22/2d847b1 f-b5c6-4a4c-8b9a-32eddf088a36.pdf [retrieved on 2012-11-20]
- CALABRÒ M L ET AL: "Effects of alpha- and beta-cyclodextrin complexation on the physico-chemical properties and antioxidant activity of some 3-hydroxyflavones", JOURNAL OF PHARMACEUTICAL AND BIOMEDICAL ANALYSIS, NEW YORK, NY, US , vol. 35, no. 2 16 April 2004 (2004-04-16), pages 365-377, XP002671789, ISSN: 0731-7085, DOI: 10.1016/J.JPBA.2003.12.005 Retrieved from the Internet: URL:http://www.sciencedirect.com/science/a rticle/pii/S0731708503007003 [retrieved on 2004-01-28]

## Description

### TECHNICAL FIELD

The present invention relates to an anti-degradation agent, and more particularly, to an anti-degradation agent which is also useful as a keeping property-improving agent for various products such as foods and cosmetics.

### BACKGROUND ART

In the course of deterioration of substances, there occur various kinds of quality changes as well as various reactions between components thereof in association with the quality changes. Besides, many of these deterioration processes are caused at the same time or in a chain-like or serial manner. As a result of recent studies, it has been obvious that most of these deterioration processes have a close relation to oxidation or photo-deterioration. It is known that the oxidation or the photo-deterioration is caused in air, in water and at an interface between air and water, between water and oil or between air and oil. It is also known that factors for accelerating the oxidative deterioration include enzymes, metals and sensitizers, and the oxidative deterioration is caused owing to combination of these factors. On the other hand, the photo-deterioration is caused when substances absorb an ultraviolet light, a visible light or a near infrared ray.

The beverages or foods and the perfumes or cosmetics tend to be usually deteriorated by oxidation of perfume components, pigment or other materials blended therein during the production process or storage thereof. Therefore, it is important to prevent such an oxidative deterioration for maintaining a good quality of each of the beverages or foods and the perfumes or cosmetics. For this reason, in the foods and the perfumes or cosmetics, there have been used, for example, natural antioxidants, synthetic antioxidants or preparations obtained by appropriately blending these antioxidants with each other (hereinafter totally referred to merely as "antioxidants").

For example, there are generally known antioxidants made of rosmaric acid, anti-degradation agents made of carnosic acid, antioxidants made of herb such as rosemary, and antioxidants containing vitamin C or vitamin E. However, these antioxidants tend to be unsatisfactory in stability of an oxidation-inhibiting performance thereof against outside environmental conditions.

In particular, it has been strongly required in application fields such as foods and perfumes or cosmetics to provide antioxidants which can exhibit a good effect even when added in a small amount and are free from deterioration even upon heating. However, at present, such antioxidants fully satisfying these requirements have not been attained until now. Further, since the photo-deterioration is sometimes caused by photo-reaction but frequently caused due to a different mechanism from that of the oxidative deterioration, the antioxidants may fail to exhibit a satisfactory effect as a photo-deterioration inhibitor even though they have a suitable oxidation preventing property. In addition, the antioxidants might sometimes undergo photo-deterioration by themselves and, therefore, may fail to exhibit a fully satisfactory effect as the photo-deterioration inhibitor.

On the other hand, it has been positively attempted to prevent the oxidation by packaging without using the antioxidants. For example, vacuum-cooked foods obtained by vacuum packaging raw foodstuffs in a bag and heat-cooking the foodstuffs while being kept in the bag at a low temperature have been noticed because such cooked foodstuffs are substantially free from dissipation of flavor and taste as well as oxidation, and inherent characteristics of the raw foodstuffs can be preserved for a long period of time. However, this method is applicable only to limited materials, and has unsolved problems concerning residual fungi, thereby still failing to be used for general purposes.

Also, in the application field of perfumes or cosmetics, in order to present deterioration of perfumes and other materials, there have been conventionally used butyl hydroxytoluene (BHT), butyl hydroxyanisole (BHA), etc. However, the use of these compounds tends to be avoided because they are synthetic chemical substances.

On the other hand, pigments have been used for tinting beverages or foods, perfumes and cosmetics, etc. In particular, for the purpose of tinting the beverages or foods, it has been demanded to use pigments derived from natural substances. However, the natural pigments tend to suffer from discoloration. In order to prevent the pigments from suffering from discoloration such as color fading and browning, there have been conventionally proposed several methods. For example, there have been proposed the color fading-preventing agent for anthocyanin-based pigments utilizing an anti-oxidative property of chlorogenic acid, caffeic acid, etc., (for example, refer to Patent Document 1); the method of preventing color fading of anthocyanin-based pigment-containing beverages and foods (for example, refer to Patent Document 2) and paprika pigments (for example, refer to Patent Document 3); the method of preventing browning of sugars by using caffeic acid, ferulic acid, chlorogenic acid, etc., (for example, refer to Patent Document 4); the method for producing browning-free candies by utilizing a browning-preventing effect of sugars (for example, refer to Patent Document 5); or the like. Further, there have been proposed antioxidants containing proanthocyanidin oligomer sampled from plants such as strained lees or seeds of grapes as an effective ingredient (for example, refer to Patent Documents 6 and 7).

The above conventionally-proposed anti-oxidative substances such as chlorogenic acid, caffeic acid and ferulic acid exhibit a relatively high effect of preventing deterioration of foods upon heating, but are not necessarily sufficient in effect of preventing deterioration of foods upon irradiation with light. On the contrary, the proanthocyanidin oligomer used for the similar purpose has the effect of preventing deterioration of foods upon irradiation with light, but is not necessarily sufficient in effect of preventing deterioration of foods upon heating. Therefore, it has been demanded to develop further improved methods.

Further, it has been reported that when an antioxidant composition composed of (a) at least one anti-oxidative substance selected from the group consisting of chlorogenic acid, caffeic acid, ferulic acid and an extract of raw coffee beans and (b) at least one component selected from the group consisting of an amino acid, a Maillard reaction product and a peptide is added to, for example, beverages or foods, perfumes or cosmetics, etc., these products can be remarkably prevented from suffering from deterioration in qualities thereof such as deterioration of perfumes, change in aroma, color fading of pigments, occurrence of strange taste or odor in foods, owing to a drastic synergistic effect which can never be achieved only by the effects of the individual components (for example, refer to Patent Document 8). However, such an antioxidant composition also has failed to exhibit a sufficient preventing effect similarly to the conventional substances and methods described previously.
Patent Document 1: Japanese Patent Publication (KOKOKU) No. 1-22872
Patent Document 2: Japanese Patent Application Laid-open (KOKAI) No. 1-132344
Patent Document 3: Japanese Patent Publication (KOKOKU) No. 59-50265
Patent Document 4: Japanese Patent Application Laid-open (KOKAI) No. 57-115147
Patent Document 5: Japanese Patent Publication (KOKOKU) No. 58-32855
Patent Document 6: Japanese Patent Publication (KOKOKU) No. 3-7232
Patent Document 7: Japanese Patent Application Laid-open (KOKAI) No. 3-200781
Patent Document 8: Japanese Patent Application Laid-open (KOKAI) No. 9-221667

WO 2004/020552 A1 discloses radical reaction inhibitors containing as the active ingredient cyclic tetrasaccharides or mixtures of cyclic tetrasaccharides with saccharide derivatives thereof, a method for inhibiting the formation of free radicals from unsaturated compounds and radical reactions of the compounds, and the compositions which contain the inhibitors and which are suppressed in radical formation, radical reactions, or progress of both.

EP 1 183 954 A1 discloses an anti-fading agent for a pigment comprising as an active ingredient at least one oligosaccharide selected from the group consisting of nigerooligosaccharide, maltooligosaccharide and panose.

JP 11 276114 A discloses a food raw material that is used as a raw material for cooked processed foods and that has a starch aging-preventing function and/or a protein denaturation-preventing function without deteriorating the tastes, flavors and textures of the foods and comprises ≤15 wt.% of maltose and ≥75 wt.% of maltotriose as solid contents.

US 5,866,179 discloses a process for the preparation of medicated chewing gums.

Hisashi Uedaira et al have discussed in the Bulletin of Chemical society of Japan, (vol. 63, no. 12, 1 January 1990, pages 3376-3379 - "Hydration of oligosaccharides") that the hydration of sugars can be determined by their conformation, and they further considered the thermodynamic properties of sugars.

CALAMBRO M L ET AL have considered effects of α- and β-cyclodextrin complexation on the physico-chemical properties and antioxidant activity of some 3-hydroxyflavones (Journal of Pharmaceutical and Biomedical Analysis, New York, NY, US, 35, 2004, 365-377).

US 2004/131709 A1 discloses compositions comprising a Labiatae herb extract and a hop extract containing beta acids and methods of using them to extend the color life and retard the growth of microorganisms in fresh meat, fish and poultry stored in an atmosphere that contains 20% or more oxygen.

US 2004/047957 A1 discloses that the color life of modified atmosphere packaged fresh red meat is extended by contacting the fresh red meat with an extract of a Labiatae herb prior to packaging the meat.

### DISCLOSURE OF THE INVENTION

### Problem to be Solved by the Invention

The present invention has been made for solving the above conventional problems. An object of the present invention is to provide an anti-degradation agent having an excellent anti-degradation performance for foods, cosmetics, etc.

### Means for Solving Problem

As a result of the present inventors' earnest study for solving the above conventional problems, it has been found that an anti-degradation agent as defined in claim 1 can exhibit an excellent effect of preventing deterioration of substances. The present invention has been attained on the basis of the above finding.

The present invention relates to:
(1) An anti-degradation agent comprising maltotriose; wherein the anti-degradation agent comprises rosmaric acid and/or vitamin C; and wherein the anti-degradation agent further comprises carnosol and carnosic acid.
(2) The anti-degradation agent as described in the above aspect, further comprising vitamin E.
(3) A food comprising the anti-degradation agent as described in any one of the above aspects.
(4) A cosmetic comprising the anti-degradation agent as described in any one of the above aspects.
(5) A glaze agent comprising the anti-degradation agent as described in any one of the aspects.
(6) A plastic product comprising the anti-degradation agent as described in any one of the aspects.

### EFFECT OF THE INVENTION

The anti-degradation agent of the present invention has a high safety, and is excellent in deterioration-preventing property for foods, cosmetics, etc.

### PREFERRED EMBODIMENTS FOR CARRYING OUT THE INVENTION

The present invention is described in detail below. The embodiments described below are only typical examples of the present invention, and not intended to limit the scope of the present invention.

### (Dynamic hydration number)

First, the concept of the "dynamic hydration number" used in the present invention is explained. It is considered that the initial reaction of deterioration is caused at an "interface", i.e., any of interfaces between gas and liquid, between liquid and solid and between liquid and liquid. In the case of almost all of foods, it is considered that the initial reaction of deterioration thereof is caused at an interface between oil and water. Here, if consideration is mainly made on the case where a configuration of the food is an aqueous system, water is generally classified into two types, i.e., liberated water and combined water. It is considered that among these waters, the liberated water frequently constitutes a factor relating to the initial reaction of deterioration.

The control of the liberated water can be realized by constraining a free motion of water molecules. The "dynamic hydration number" of the compound is an index showing a degree of constraint of free motion of water when the compound is dissolved in water. From the physical viewpoint, the dynamic hydration number represents an amount of a relative change in "thermal motion of water molecules in bulk water" based on "thermal motion of whole water molecules in a hydration phase" assuming that the numbers of water molecules in the bulk water and the hydration phase are identical to each other (reference literature: Uedaira, H, "Bull. Chem. Soc., Jpn.", Vol. 62, No. 1 (1989)). The dynamic hydration number of the compound may be readily measured, for example, by the following method.

### (Experimental example for measurement of dynamic hydration number)

25.0 mg of p-hydroxybenzoic acid was dissolved in pure water, and pure water was further added the resultant solution to obtain 50 mL of a solution (blank). The blank was added to samples used in amounts of 1.008 g and 1.512 g, respectively, to prepare sample solutions 1 and 2 having total weights of 4.013 g and 3.001 g, respectively. Using a spectrophotometer "Inova 500" manufactured by Varian Corp., the relaxation time (T1) of the respective sample solutions was measured at 25°C to determine a dynamic hydration number thereof. An example of the results is shown in the following Table 1.

**Table 1**

| (Dynamic hydration number of various kinds of compounds: at 25°C) | |
|---|---|
| Compounds | Dynamic hydration number (nDHN) |
| Xylose | 15.5 |
| Fructose | 16.5 |
| Glucose | 18.6 |
| Lysine | 3.0 |
| Valine | 21.0 |
| Sucrose | 15.0 |
| Maltotriose ("OLIGOTOSE (tradename)" produced by Mitsubishi-Kagaku Foods Corporation) | 204 |
| Maltotriol ("OLIGOTOSE H-70 (tradename)" produced by Mitsubishi-Kagaku Foods Corporation) | 211 |

As the dynamic hydration number increased, the motion of a larger amount of water can be constrained. Therefore, it is expected that when the dynamic hydration number is large, liberated water as a degradation factor can be well controlled. More specifically, when adding a substance (compound) having a large dynamic hydration number to water, the amount of liberated water contained in water is decreased. As a result, it is considered that the degradation factor generated in water is concentrated or localized to an interface thereof with which the substance added to water cannot be associated.

### <Anti-degradation agent (I)>

First, the anti-degradation agent (I) according to the one aspect of the present disclosure is explained. The anti-degradation agent (I) is characterized by comprising a dynamic hydration number of not less than 25.

This aspect of the present disclosure has been found on the basis of the above finding, in which the compound having a dynamic hydration number of not less than 25 is added to the products to efficiently prevent the degradation factor from being concentrated or localized to an interface thereof. Examples of the compound having a dynamic hydration number of not less than 25 may include sugars such as maltotriose, sugar alcohols such as maltotriol, proteins and peptides. The anti-degradation agent of the present invention comprises maltotriose.

### <Anti-degradation agent (II)>

Next, the anti-degradation agent (II) according to the second aspect of the present disclosure is explained. The anti-degradation agent (II) is characterized by comprising a tri- or higher oligosaccharide, a polysaccharide or a derivative thereof.

The trisaccharide is a compound obtained by bonding three monosaccharide molecules to each other. The tri- or higher oligosaccharide means a compound obtained by bonding about 3 to 20 monosaccharide molecules to each other. The compound obtained by bonding a still larger number of monosaccharide molecules to each other is referred to as a polysaccharide. The oligosaccharide is readily dissolved in water, whereas the polysaccharide is hardly dissolved in water. Examples of the derivatives of the tri- or higher oligosaccharide and the polysaccharide may include deoxy sugars obtained by replacing a hydroxyl group of sugars with hydrogen, uronic acids obtained by replacing carbon bonded to a terminal end of aldoses with a carboxyl group, amino sugars obtained by replacing a hydroxyl group of sugars with an amino group, and sugar alcohols obtained by reducing a ketone group or an aldehyde group of sugars to an alcohol group.

Examples of the oligosaccharide may include isomalto-oligosaccharides, fructo-oligosaccharides, xylo-oligosaccharides, soybean oligosaccharides and maltotriose. Examples of the polysaccharide may include starch, amylose, amylopectin, glycogen (popular name: animal starch), cellulose, chitin, agarose, carageenan, pectin, heparin and hyaluronic acid.

Examples of the low-molecular weight sugar alcohols may include xylitol, sorbitol, manitol, multitol and lactitol. These compounds are capable of forming constitutional units of the derivatives (sugar alcohols) of the tri- or more oligosaccharide and the polysaccharide. Meanwhile, the sugar alcohol obtained by subjecting maltotriose as the tri- or more oligosaccharide to reduction reaction is maltotriol. The anti-degradation agent according to the invention comprises maltotriose. The maltotriose is a linear oligosaccharide having a polymerization degree of not more than 3 and exhibiting a highest moisture retention property among the oligosaccharides, and the degree of sweetness of the maltotriose is as low as about one-fourth time that of sugar. For this reason, the maltotriose can enhance a sugar content without increase in the sweetness degree and, therefore, can show a high safety and a low degree of sweetness event when added in a large amount. Further, when adding a large amount of the maltotriose to the products, it is possible to exhibit various functions such as antiseptic property and anti-aging property for starches. As the readily available maltotriose, there may be exemplified the commercial product "OLIGOTOSE" produced by Mitsubishi-Kagaku Foods Corporation. Meanwhile, the oligosaccharide alcohol obtained by subjecting the "OLIGOTOSE" to reduction reaction is commercially available under the tradename "OLIGOTOSE H-70" from Mitsubishi-Kagaku Foods Corporation.

### <Combinations of anti-degradation agents (I) and (II) with third components>

When adding the respective anti-degradation agents (I) and (II) of the present disclosure and invention in combination with an adequate third component, the anti-degradation property thereof can be further enhanced.

In general, the oil-insoluble compound means such a compound having substantially no solubility in natural fats and oils such as vegetable oils, i.e., a solubility in triglyceride of not more than 0.1%. However, the solubility in triglyceride of the compound varies depending upon constitutional fatty acids of the triglyceride. Therefore, in the present disclosure, the oil insolubility and oil solubility of the compound is defined by a solubility thereof in hexane. The oil solubility (solubility in hexane) of the main antioxidants are shown in Table 2 below.

**Table 2**

| (Solubility in hexane: g/L (at 30°C)) | | |
|---|---|---|
| Water-soluble rosemary extract (tradename "RM21A base" produced by Mitsubishi-Kagaku Foods Corporation) | 0.1 | Oil-insoluble |
| Water-insoluble rosemary extract (tradename "RM21B base" produced by Mitsubishi-Kagaku Foods Corporation) | 10 | Oil-insoluble |
| Pinophenone | 25 | Oil-insoluble |
| Teacaron | 8 | Oil-insoluble |
| Vitamin C (ascorbic acid) | 0.1 | Oil-insoluble |
| Sunmerry | 1 | Oil-insoluble |
| Vitamin E (tocopherol) | >500 | Oil-soluble |

First, the kinds of additive components used in the anti-degradation agents (I) and (II) of the present disclosure are explained.

### (Additive component-1)

The additive component-1 of the present disclosure is an oil-insoluble antioxidant having a solubility in 1 L of hexane of less than 50 g as measured at 30°C. Such an oil-insoluble antioxidant is classified into the below-mentioned additive component-2 and additive component-3.

As described above, it is considered that when adding the substance (compound) having a large dynamic hydration number into water, the deterioration factor generated in water is concentrated or localized to the interface with which the substance added into water cannot be associated. Therefore, when the anti-degradation agents are added to the position where the deterioration factor tends to be concentrated or localized, i.e., at the portion of liberated water which is not constrained by the substance added or at the interface thereof, it is considered that the action of the deterioration factor concentrated or localized to the portion of liberated water or the interface thereof can be effectively inhibited.

Many of foods are composed of oil and water and, therefore, hardly dissolved in oils. More specifically, it is expected that the oil-insoluble antioxidant added basically exists in water or at the interface between water and oil. The oil-insoluble antioxidant being present in water directly acts on the liberated water being not controlled. That is, from the standpoint of a water solubility, such an antioxidant as the additive component-1 is further classified into the following two groups.

### (Additive component-2)

The additive component-2 is an oil-insoluble and water-soluble antioxidant having a solubility in 1 L of hexane of less than 50 g as measured at 30°C and a solubility in 100 g of water of not less than 0.1 g as measured at 25°C.

Examples of the above oil-insoluble and water-soluble antioxidant may include, in addition to rosmaric acid, vitamin C (ascorbic acid), isoascorbic acid and salts thereof (such as, for example, alkali metal salts and alkali earth metal salts), and water solubility-imparted catechins (such as, for example, glycosides and dextrin-enclosed products thereof).

Examples of ascorbic acid and salts thereof may include L-ascorbic acid, sodium L-ascorbate, erythorbic acid and sodium erythorbate. Examples of the above natural extracts may include rutin, myricetin, myricitrin, chlorogenic acid, caffeic acid, ferulic acid, gallic acid, water-soluble rosemary extracts (such as, for example, "RM21A base (tradename)" produced by Mitsubishi-Kagaku Foods Corporation), tea extracts (such as, for example, "TEACARON" produced by TOKIWA PHYTOCHEMICAL CO.,LTD.), apple extracts, grape seed extracts (such as, for example, "PINOPHENONE (tradename)" produced by TOKIWA PHYTOCHEMICAL CO.,LTD.), bayberry extracts (such as, for example, "SUNMERRY (tradename)" produced by San-Ei Gen F.F.I.,Inc.), sunflower seed extracts, and rice bran extracts. Among these substances, especially preferred are rosmaric acid and/or vitamin C. The anti-degradation agent of the present invention comprises rosmaric acid and/or vitamin C.

The rosmaric acid is one of phenol-carboxylic acids contained in herbs, in particular, the rosmaric acid is contained in a large amount in rosemary. The rosmaric acid has such a structure in which two phenol-carboxylic acids are bonded to each other. Therefore, the rosmaric acid structurally and functionally exhibits a higher oxidation-preventing effect than those of phenol-carboxylic acids such as ferulic acid, caffeic acid and chlorogenic acid because the rosmaric acid contains a larger number of phenolic hydroxyl groups. Further, the rosmaric acid exhibits a high effect of activating inhibition of enzyme reaction like SOD (superoxide dimustase). In addition, the rosmaric acid also has a high photo-deterioration inhibiting effect because of conjugated double bond contained in its structure.

The rosmaric acid used in the present invention is preferably in the form of a natural extract from the viewpoint of a high safety. Also, the rosmaric acid used in the present invention includes the glycosides of rosmaric acid. The glycosides having any structure may be used in the present invention. Natural products of the rosmaric acid may be in the form of an extract obtained from herbs, in particular, Lamiacea plants and preferably extracts obtained from rosemary containing a large amount of rosmaric acid.

The general method for producing rosmaric acid is as follows. As the raw material, there may be used a whole grass of rosemary, or any of leaves, roots, stems, flowers, fruits and seeds of rosemary. Among them, preferred are leaves of rosemary. In general, in order to enhance the extraction efficiency, rosemary may be used in the form of cut pieces. The rosmaric acid is obtained in the form of a water-soluble extract of rosemary. Therefore, the rosmaric acid may be produced by subjecting rosemary to extraction treatment with hydrated ethanol, adding water to the resultant extract to precipitate water-insoluble components therefrom, and concentrating the obtained solution from which the water-insoluble components are removed, under reduced pressure. As the hydrated ethanol, there may be suitably used those having a water content of 40 to 60% by weight.

Further, when adding a water-insoluble anti-degradation agent, the agent is concentrated or localized to the interface, thereby inhibiting the action of the deterioration factor concentrated or localized to the above-mentioned controlled liberated water, uncontrolled liberated water and other interfaces. Such a water-insoluble anti-degradation agent is classified into the below-mentioned additive component-3 and additive component-4.

### (Additive component-3)

The additive component-3 is an oil-insoluble and water-insoluble antioxidant having a solubility in 1 L of hexane of less than 50 g as measured at 0°C and a solubility in 100 g of water of less than 0.1 g as measured at 25°C. The solubility in water of the additive component-3 is preferably not more than 0.05 g and more preferably not more than 0.01 g.

Examples of the oil-insoluble and water-insoluble antioxidant may include catechin, epicatechin, epigallocatechin, catechin gallate, epigallocatechin gallate, vitamin C fatty esters, water-insoluble rosemary extracts (such as, for example, "RM21B base (tradename)" produced by Mitsubishi-Kagaku Foods Corporation), carnosol and carnosic acid. Among these oil-insoluble and water-insoluble antioxidants, preferred are carnosol and/or carnosic acid. The anti-degradation agent of the present invention comprises carnosol and carnosic acid.

The carnosol and carnosic acid are contained in a large amount in herb-based condiments such as sage, thyme and oregano. The carnosol and carnosic acid have an abietane structure containing an isoprene skeleton unlike the other antioxidants and, therefore, exhibit a considerably high oxidation-preventing effect on fats and oils, etc., as compared to the other antioxidants. In addition, the carnosol and carnosic acid have a conjugated double bond in a structure thereof and further a tautomerism structure. Owing to the tautomerism structure, the carnosol and carnosic acid tend to have a radical stabilized structure even when undergoing influence of radicals produced by exposure to light and, therefore, exhibit a high photo-deterioration preventing effect.

The carnosol and carnosic acid used in the present invention are preferably in the form of a natural extract from the viewpoint of a high safety. Natural products of the carnosol and carnosic acid may be in the form of an extract obtained from herb-based plants such as sage, thyme and oregano. Among these extracts, preferred are extracts obtained from rosemary containing a large amount of carnosol and carnosic acid.

The carnosol and carnosic acid may be obtained as a water-insoluble extract of rosemary. An example of the method for producing the carnosol and carnosic acid is as follows. First, similarly to the above water-soluble extracts, rosemary is subjected to extraction treatment with hydrated ethanol, adding water to the resultant extract to precipitate water-insoluble components therefrom. After the resultant mixture is mixed with activated carbon and then stirred, a mixture of the water-insoluble components and activated carbon is separated therefrom. The resultant mixture is further subjected to extract treatment with ethanol, and the obtained extract was distilled to remove ethanol therefrom, thereby obtaining the carnosol and carnosic acid in the form of a powdered concentrate. The details of the above method for production of the carnosol and carnosic acid will become more apparent by referring to descriptions of Japanese Patent Publication (KOKOKU) No. 59-4469.

### (Additive component-4)

The additive component-4 is an oil-soluble antioxidant having a solubility in 1 L of hexane of not less than 50 g as measured at 30°C. Examples of the oil-soluble antioxidant may include tocotrienol, ubiquinone (coQ10), rice oil extracts, vitamins E (α-, β-, γ- and δ-tocopherol) and mixed tocopherol. Among these oil-soluble antioxidants, preferred is vitamin E (including mixed tocopherol).

### (Additive component-5)

In the present invention, the anti-degradation agent may also be used in combination with as a further component, for example, polyglycerol fatty esters such as polyglycerol laurate, polyglycerol myristate, polyglycerol palmitate, polyglycerol stearate and polyglycerol oleate; and sucrose fatty esters such as sucrose laurate, sucrose myristate, sucrose palmitate, sucrose stearate and sucrose oleate.

Next, the amounts of the above additive components used are explained.

### (In the case of anti-degradation agent (I))

When the above additive components are used in combination with the compound having a dynamic hydration number of not less than 25, the content of the compound having a dynamic hydration number of not less than 25 in the anti-degradation agent (I) is usually 20 to 99% by weight, preferably 20 to 80% by weight and more preferably 20 to 60% by weight. The compound having a dynamic hydration number of not less than 25 is preferably used in combination with the additive component-1 (oil-insoluble antioxidant), in particular, with the additive component-2 (oil-insoluble and water-soluble antioxidant). As described above, as the additive component-2, rosmaric acid and/or vitamin C are used in the anti-degradation agent of the invention. The optimum content of the additive component-2 in the anti-degradation agent (I) varies depending upon kind thereof. In general, the content of the additive component-2 in the anti-degradation agent (I) is usually 0.01 to 50% by weight, preferably 0.05 to 30% by weight and more preferably 0.1 to 30% by weight. In particular, when using rosmaric acid as the additive component-2, the content of rosmaric acid in the anti-degradation agent (I) is usually 0.1 to 20% by weight, preferably 0.1 to 10% by weight and more preferably 0.2 to 5% by weight. When the content of rosmaric acid in the anti-degradation agent (I) is too small, the resultant anti-degradation agent tends to be deteriorated in oxidation-preventing property at the interface between water and oil. When the content of rosmaric acid in the anti-degradation agent (I) is too large, the resultant anti-degradation agent tends to be adversely affected by photo-catalyzed metals. Also, the weight ratio of the oil-insoluble and water soluble antioxidant to the compound having a dynamic hydration number of not less than 25 is usually from 1/1000 to 1/1, preferably from 1/500 to 1/5 and more preferably from 1/200 to 1/10.

Further, the anti-degradation agent of the invention comprises as the additive component-3 (oil-insoluble and water-insoluble antioxidant) carnosol and carnosic acid. The content of the additive component-3 in the anti-degradation agent (I) is usually 0.05 to 10% by weight, preferably 0.05 to 3% by weight and more preferably 0.1 to 1% by weight. The weight ratio between the oil-insoluble and water-soluble antioxidant, the compound having a dynamic hydration number of not less than 25 and a sum of carnosol and carnosic acid (oil-insoluble and water-soluble antioxidant/compound having a dynamic hydration number of not less than 25/sum of carnosol and carnosic acid) is usually from 1/1000/0.1 to 1/1/10, preferably from 1/500/0.1 to 1/5/10 and more preferably from 1/200/0.1 to 1/10/10.

In the anti-degradation agent of the present invention, there is preferably used vitamin E. The content of vitamin E and the weight ratio thereof to the other components are the same as those for the additive component-3.

In addition, the amount of the additive component-5 used varies depending upon kind thereof and, therefore, is not particularly limited. Thus, the amount of the additive component-5 used may be appropriately selected from such a range that the effect of the resultant anti-degradation agent (I) is not adversely affected.

### (In the case of anti-degradation agent (II))

When the tri- or higher oligosaccharide, polysaccharide or derivative thereof as disclosed herein(these compounds are hereinafter typified by maltotriose that is used in the anti-degradation agent of the invention) is used in combination with the above additive components, the content of maltotriose in the anti-degradation agent (II) is usually 20 to 99% by weight, preferably 20 to 80% by weight and more preferably 20 to 60% by weight.

The preferred embodiments of combination of maltotriose and the above additive components in the anti-degradation agent (II) are just the same as those of the above anti-degradation agent (I). The anti-degradation agent of the invention is as defined in claim 1.

More specifically, the content of the additive component-2 in the anti-degradation agent (II) is usually 0.01 to 50% by weight, preferably 0.05 to 30% by weight and more preferably 0.1 to 30% by weight. In particular, when the additive component-2 is rosmaric acid, the content of rosmaric acid in the anti-degradation agent (II) is usually 0.1 to 20% by weight, preferably 0.1 to 10% by weight and more preferably 0.2 to 5% by weight. The weight ratio of the oil-insoluble and water-soluble antioxidant to maltotriose is usually from 1/1000 to 1/1, preferably from 1/500 to 1/5 and more preferably from 1/200 to 1/10. The content of the additive component-3 in the anti-degradation agent (II) is usually 0.05 to 10% by weight, preferably 0.05 to 3% by weight and more preferably 0.1 to 1% by weight. The weight ratio between the oil-insoluble and water-soluble antioxidant, maltotriose and a sum of carnosol and carnosic acid (oil-insoluble and water-soluble antioxidant/maltotriose/sum of carnosol and carnosic acid) is usually from 1/1000/0.1 to 1/1/10, preferably from 1/500/0.1 to 1/5/10 and more preferably from 1/200/0.1 to 1/10/10.

The anti-degradation agents of the present disclosure and invention are respectively produced by mixing the above components with each other. The order of mixing of the respective components is not particularly limited. The respective anti-degradation agents of the present disclosure and invention are usually in the form of a solution prepared by dissolving the above respective components in water or a mixed solvent comprising of ethanol and water. When using carnosol and carnosic acid in combination with the other components in the anti-degradation agents, the respective components are usually dissolved in a mixed solvent comprising ethanol and water. Such a solution is usually prepared by mixing the above respective components with each other, and thereafter adding ethanol and then water to the obtained mixture. The mixing ratio of water to ethanol is usually 1:1 to 3:1. The antioxidant used in the present invention may be in the form of a powder. The powder may be produced by spray-drying or freeze-drying the above solution.

The anti-degradation agent of the present invention is preferably applied to readily-deteriorated products such as foods and cosmetics. The amount of the anti-degradation agent added may be determined within an optimum range according to the kind of each product. In general, the amount of the anti-degradation agent added to the product is usually 0.001 to 30% by weight, preferably 0.01 to 10% by weight and more preferably 0.05 to 5% by weight on the basis of the weight of the product.

### <Products using the anti-degradation agent of the present invention>

### (Food)

As the food of the present invention, there may be suitably used those foods which tend to be readily deteriorated in quality. Specific examples of the food may include beverages, milk beverages, alcohol beverages, boiled rice, beans (such as rice, wheat, barley, corn, millet and barnyard millet), bread and other wheat flour products, noodles, roux such as curry roux and stew roux, retort foods, seasonings, dairy products such as ice cream, and diets (including pet foods).

Among the above foods, the processed marine, livestock or oil and fat products are preferably those processed marine, livestock or oil and fat products which tend to be readily deteriorated in quality or should be preserved for a long period of time. Specific examples of the processed marine, livestock or oil and fat products may include fresh fishes, dried fishes, overnight-dried fishes, dried mirin-seasoned fishes, pigment-keeping agents for shells, red-meat fishes and Crustacea, minced or ground fish meat, marine paste products, foods of delicate flavor, fish sausage, salt-preserved products, laver, seaweed products, unsaturated polyvalent fatty acids such as α-linolenic acid, docosahexaenoic acid (DHA) and eicosapentaenoic acid (EPA) and triglycerides thereof as well as foods containing thereof, chicken, pork, beef, mutton, sausage, ham and their processed products containing these compounds, corn flakes, instant Chinese noodles, oil cakes using oils and fats, fast spreads, and margarine.

The amount of the anti-degradation agent of the present invention used in the food is usually 0.001 to 30% by weight, preferably 0.01 to 10% by weight and more preferably 0.05 to 5% by weight on the basis of the weight of the food.

### (Cosmetic)

As the cosmetic of the present invention, there may be suitably used those cosmetics which tend to be readily deteriorated in quality. Specific examples of the cosmetic may include humectants, beauty white agents, cleansing solutions, lotions, detergents, softening agents, finishing agents, dish-washing agents, detergents for vegetables and fruits, and rinsing agents. The amount of the anti-degradation agent of the present invention used in the cosmetic is usually 0.001 to 30% by weight, preferably 0.01 to 10% by weight and more preferably 0.05 to 5% by weight on the basis of the weight of the cosmetic.

### (Glaze agent)

The term "glaze" means to coat the surface of landed fish with ice upon freezing the landed fish, and the glaze agent is used for forming a uniform ice coat on the surface of the landed fish. The amount of the anti-degradation agent of the present invention used in the glaze agent is usually 0.001 to 30% by weight, preferably 0.01 to 10% by weight and more preferably 0.05 to 5% by weight on the basis of the weight of the glaze agent.

### (Plastic product)

The anti-degradation agent of the present invention can be added to a plastic product to indirectly prevent beverage or foods, perfumes or cosmetics and other products packaged therein from being deteriorated in quality. Specific examples of the plastic product may include plastic containers for the beverages or foods and perfumes or cosmetics, packaging materials for foods such as baran (aspidistra) partitions and packs for preservation of cooked foods, packaging materials for sanitary goods such as deodorants and liquid detergents, domestic electrical appliances such as refrigerators, air conditioners, air cleaners and laundry dryers, and air conditioning equipments for ships, automobiles, trains, airplanes and buildings. The amount of the anti-degradation agent of the present invention used in the plastic product is usually 0.001 to 20% by weight, preferably 0.01 to 10% by weight and more preferably 0.1 to 5% by weight on the basis of the weight of the plastic product.

### EXAMPLES

The present invention is described in more detail by Examples. However, it should be noted that the following Examples are only illustrative and not intended to limit the scope of the present invention.

### Production Example 1: (Production of water-soluble extract of rosemary)

10 L of 50% hydrated ethanol was added to 1 kg of rosemary, and the resultant mixture was refluxed under heating for 3 hr and then filtered under a hot condition, thereby obtaining a filtrate. The obtained residue was extracted with 6 L of 50% hydrated ethanol, and this extraction procedure was further repeated two times, thereby obtaining filtrates. The obtained filtrates were combined together and mixed with 5 L of water to form a precipitate. The resultant solution was mixed with 100 g of activated carbon, stirred for 1 hr, allowed to stand in a cold place overnight, and then filtered to obtain a filtrate. The thus obtained filtrate was concentrated under reduced pressure, thereby obtaining 120 g of a water-soluble rosemary extract (rosmaric acid content: 31.6% by weight).

### Production Example 2: (Production of water-insoluble extract of rosemary)

10 L of 50% hydrated ethanol was added to 1 kg of rosemary, and the resultant mixture was refluxed under heating for 3 hr and then filtered under a hot condition, thereby obtaining a filtrate. The obtained residue was extracted with 6 L of 50% hydrated ethanol, and this extraction procedure was further repeated two times, thereby obtaining filtrates. The obtained filtrates were combined together and mixed with 5 L of water to form a precipitate. The resultant filtrate was mixed with 100 g of activated carbon, stirred for 1 hr, allowed to stand in a cold place overnight, and then filtered to obtain a mixture of the precipitate and the activated carbon. The thus obtained mixture was mixed with 4 L of ethanol, refluxed under heating for 3 hr, and then filtered under a hot condition, thereby obtaining a filtrate. The obtained residue was extracted with 2.4 L of ethanol, and this extraction procedure was further repeated two times, thereby obtaining filtrates. The obtained filtrates were combined together and concentrated under reduced pressure to distil off ethanol therefrom, thereby obtaining a water-insoluble powdery rosemary extract (total content of carnosol and carnosic acid: 24.9% by weight).

### Examples 1 to 13 (Examples 1-3, 7-8 and 9-12 are reference examples) and Comparative Examples 1 to 6: (Anti-degradation test for mackerel)

A mackerel was subjected to anti-degradation test according to the following procedure. First, a mackerel prepared was opened, and the surface of the opened mackerel was washed with water. Then, the mackerel was immersed in 2L of a 10wt% saline containing 20 mL of the anti-degradation agent as the test sample. After being immersed, the mackerel was washed again with water, and then dried at 25°C for 2 hr. After being dried, the mackerel was preserved at 5°C for 8 days to visually observe the condition of deterioration of the respective mackerels. The evaluation was made according to the ratings shown in Table 3.

**Table 3**

| Rating | Reason for evaluation rating | Commercial value |
|---|---|---|
| ⊚ | Substantially no change from initial condition. Excellent commercial value was recognized by experts. (High motivation to buy the anti-degradation agent used ) | Superfine grade |
| ○ | Substantially no change from initial condition. Good commercial value was recognized by experts. | First grade |
| ○Δ | Slight deterioration from initial condition, but Good commercial value was still recognized by experts. | Quasi-first grade |
| Δ | Apparent deterioration from initial condition, but certain commercial value was still recognized. | Second grade |
| Δ× | Apparent deterioration from initial condition, and hardly usable as a commercial product. | No commercial value |
| × | Apparent deterioration from initial condition, and no commercial value was recognized. | No commercial value |

The respective components shown in the following Table 4 were mixed with each other at the weight ratios as shown in Table 4. The obtained mixture was dissolved in a mixed solvent composed of 4 mL of ethanol and water as a balance to prepare 20 mL of an anti-degradation agent as a test sample. Using the thus prepared anti-degradation agent, mackerel was subjected to the above anti-degradation test. The results are shown in Table 4. In addition, the details of the respective components described in Table 4 are shown in Table 5.

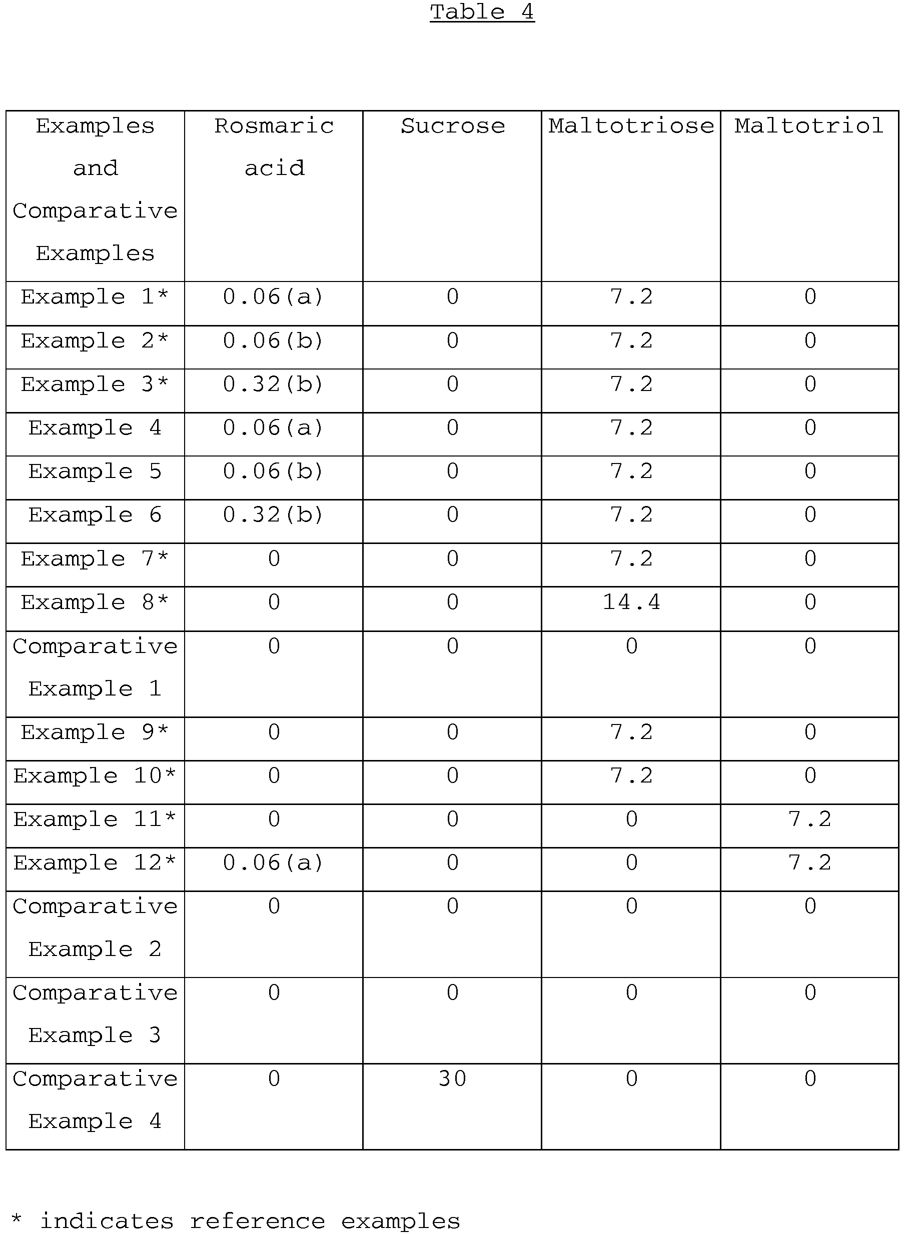

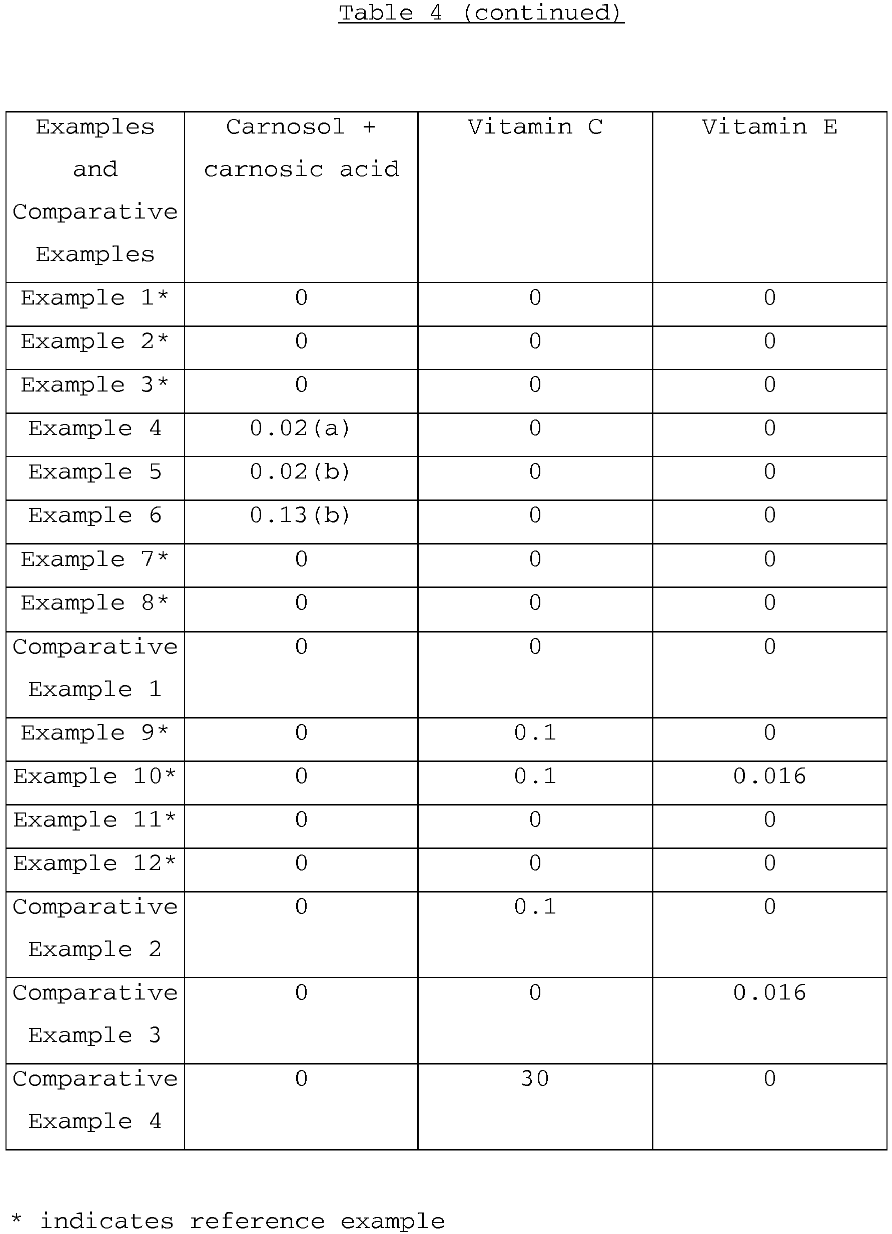

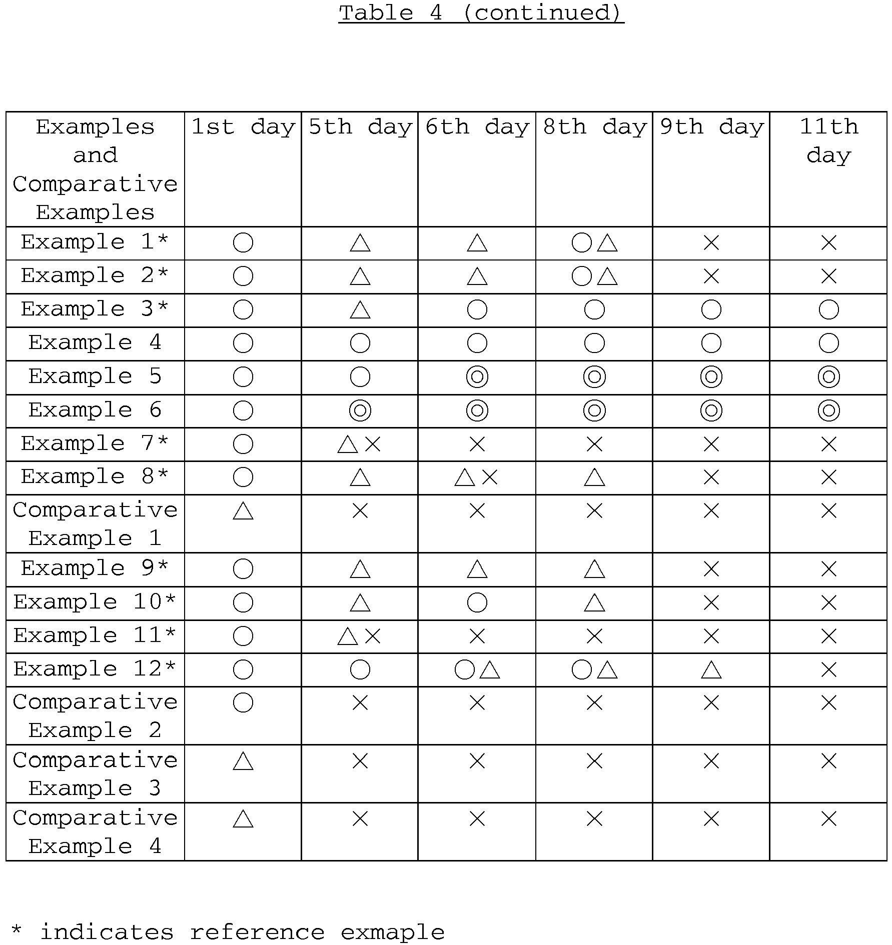

**Table 5**

| | | |
|---|---|---|
| Rosmaric acid | (a) | Commercial product produced by Merk Corp. |
| | (b) | Water-soluble extract of rosemary obtained in Production Example 1 (content of rosmaric acid: 31.6% by weight) |
| Sucrose | | Commercial product produced by Mitsubishi-Kagaku Foods Corporation |
| Maltotriose | | Commercial product "OLIGOTOSE" produced by Mitsubishi-Kagaku Foods Corporation |
| Maltotriol | | Commercial product "OLIGOTOSE H-70" produced by Mitsubishi-Kagaku Foods Corporation |
| Carnosol + carnosic acid | (a) | Commercial product (carnosol and carnosic acid) produced by Keiman Corp. |
| | (b) | Water-insoluble extract of rosemary obtained in Production Example 1 (total content of carnosol and carnosic acid: 24.9% by weight) |
| Vitamin C | | Commercial product (sodium L-ascorbate) produced by Tokyo Kasei Co., Ltd. |
| Vitamin E | | Commercial product (mixed tocopherol) produced by Amakasu-sha Co., Ltd. |

### Example 13 and Comparative Example 5: (Anti-photo-degradation test for milk)

Milk was subjected to anti-photo-degradation test according to the following procedure. That is, 1 mL of the anti-degradation agent obtained in Example 6 was added to 100 mL of milk to prepare a test sample (Example 13). Whereas, 100 mL of milk containing no anti-degradation agent was prepared as a comparative test sample (Comparative Example 5). Both of the test samples were irradiated with light having a total luminous amount of 20000 luxes for 48 hr at 5°C. Thereafter, using a headspace gas chromatography "Agilent 6890GC", the amount of hexanal as a volatile component in the respective test samples was measured to compare a compositional area ratio of hexanal in the test sample containing the anti-degradation agent with that of the comparative test sample containing no anti-degradation agent, thereby evaluating the degree of degradation of milk. The degree of degradation of milk was expressed in terms of the ratio based on that of the comparative sample containing no anti-degradation agent as 100. The results are shown in Table 6.

**Table 6**

| | Amount of hexanal after irradiation with light (area ratio) |
|---|---|
| Example 13 | 0.1 |
| Comparative Example 5 | 100 |

From the results of the above Examples 1 to 13, it was apparently confirmed that the anti-degradation agent of the present invention was effective for preventing oxidation deterioration and photo-deterioration.

## Claims

1. An anti-degradation agent comprising maltotriose; wherein the anti-degradation agent comprises rosmaric acid and/or vitamin C; and wherein the anti-degradation agent further comprises carnosol and carnosic acid.

2. The anti-degradation agent according to claim 1, further comprising vitamin E.

3. A food comprising the anti-degradation agent as defined in any one of claims 1 to 2.

4. A cosmetic comprising the anti-degradation agent as defined in any one of claims 1 to 2.

5. A glaze agent comprising the anti-degradation agent as defined in any one of claims 1 to 2.

6. A plastic product comprising the anti-degradation agent as defined in any one of claims 1 to 2.

## Patentansprüche

1. Antizersetzungsmittel, umfassend Maltotriose, wobei das Antizersetzungsmittel Rosmarinsäure und/oder Vitamin C umfasst; und wobei das Antizersetzungsmittel ferner Carnosol und Carnosolsäure umfasst.

2. Antizersetzungsmittel gemäß Anspruch 1, ferner umfassend Vitamin E.

3. Nahrungsmittel, umfassend das wie in einem der Ansprüche 1 bis 2 definierte Antizersetzungsmittel.

4. Kosmetikum, umfassend das wie in einem der Ansprüche 1 bis 2 definierte Antizersetzungsmittel.

5. Glasurmittel, umfassend das wie in einem der Ansprüche 1 bis 2 definierte Antizersetzungsmittel.

6. Kunststoffprodukt, umfassend das wie in einem der Ansprüche 1 bis 2 definierte Antizersetzungsmittel.

## Revendications

1. Agent anti-dégradation comprenant du maltotriose ; dans lequel l'agent anti-dégradation comprend de l'acide rosmarique et/ou de la vitamine C ; et dans lequel l'agent anti-dégradation comprend en outre du carnosol et de l'acide carnosique.

2. Agent anti-dégradation selon la revendication 1, comprenant en outre de la vitamine E.

3. Aliment comprenant l'agent anti-dégradation tel que défini dans l'une quelconque des revendications 1 à 2.

4. Produit cosmétique comprenant l'agent anti-dégradation tel que défini dans l'une quelconque des revendications 1 à 2.

5. Agent de glaçage comprenant l'agent anti-dégradation tel que défini dans l'une quelconque des revendications 1 à 2.

6. Produit plastique comprenant l'agent anti-dégradation tel que défini dans l'une quelconque des revendications 1 à 2.
